# EUROPEAN PATENT APPLICATION

(11) **EP 3 895 669 A1**
(43) Date of publication of application: **20.10.2021**
(21) Application number: 21166647.4
(22) Date of filing: 01.04.2021
(51) Int. Cl.: A61F 2/95, A61F 2/97

(54) **PRELOADED DELIVERY SYSTEM FOR FENESTRATED OR BRANCHED STENT GRAFT**

(30) Priority: 06.04.2020 US 202063005631 P
(71) Applicant: Cook Medical Technologies LLC, Bloomington, IN 47404 (US)
(72) Inventor: WILGER, Kevin D., Lafayette, IN 47905 (US); KRATZBERG, Jarin A., West Lafayette, IN 47906 (US); BREDWAY, Ryan C., Lafayette, IN 47901 (US)
(74) Representative: Warren, Caroline Elisabeth

(57) **Abstract**

The disclosure is directed to a pre-loaded stent graft delivery device. The pre-loaded stent graft delivery device includes a guide wire catheter having a guide wire lumen therethrough; and a handle assembly disposed at a distal end of the guide wire catheter. The handle assembly includes a proximal handle, a rotational handle, a slot, and a sheath follower. The proximal handle, including a manifold including at least one side port, is disposed at a proximal end of the handle assembly. The slot is disposed inside the rotational handle, and is fixed and remains stationary relative to the proximal handle. The sheath follower is disposed slidably in the slot and fixed to a distal portion of a sheath. The rotational handle includes an internal thread mechanically coupled with the sheath follower, so that, when the rotational handle rotates toward a first rotational direction, the sheath is pulled to move distally.

## Description

### RELATED APPLICATION

This application claims priority to Provisional Patent Application No. 63/005,631, filed on April 6, 2020, which is incorporated by reference in its entirety.

### BACKGROUND

### 1. Technical Field

The present disclosure relates to a device for use in medical operation. In particular, the present disclosure relates to a medical device for introduction or delivery of a stent graft into a vasculature of a patient.

### 2. Background Information

Endovascular repair may become necessary in cases of separation of the aorta's layers (dissection), narrowing of the aorta (stenosis), and traumatic damages of the aorta (transection). For example, endovascular repair of abdominal aortic aneurysms and thoracic aortic aneurysms are typically treated by placing a stent graft inside the affected vessels. The task of stent grafts is to keep the blood vessels open, to allow sufficient blood flow, and to prevent further vessel wall expansion, thus, lowering the risk of a detrimental vessel rupture.

Clinical procedures of placing an interventional device into a body cavity includes placing a stent graft inside an affected vessel. For example, aortic aneurysms that involve or are adjacent to critical vessels are treated with fenestrated graft devices and bridging stent grafts.

The existing delivery systems may require long sheaths and catheters to cannulate those vessels. There may be a number of disadvantages and problems associated with using long catheters, which may include that long catheters may be difficult to torque and track, and an increase of operating time and radiation exposure. The above problems may increase difficulty of performing the medical procedure.

The present disclosure is directed toward addressing one or more drawbacks, including but not limited to those set forth above.

### BRIEF SUMMARY

Aspects of the invention are set out in the independent claims and preferred features are set out in the dependent claims. Preferred features of one aspect may be applied to other aspects.

The present disclosure describes one embodiment of a pre-loaded stent graft delivery device. The pre-loaded stent graft delivery device includes a guide wire catheter having a guide wire lumen therethrough; and a handle assembly disposed at a distal end of the guide wire catheter. The handle assembly includes a proximal handle, a rotational handle, a slot, and a sheath follower. The proximal handle is disposed at a proximal end of the handle assembly. The proximal handle includes a manifold including at least one side port and a through bore. The slot is disposed inside the rotational handle, and is fixed and remains stationary relative to the proximal handle. The sheath follower is disposed slidably in the slot, and the sheath follower is configured to fix to a distal portion of a sheath. The rotational handle includes an internal thread mechanically coupled with the sheath follower, so that, when the rotational handle rotates toward a first rotational direction, the sheath is pulled to move distally.

The present disclosure describes another embodiment of a pre-loaded stent graft delivery device. The pre-loaded stent graft delivery device includes a guide wire catheter having a guide wire lumen therethrough; and a handle assembly disposed at a distal end of the guide wire catheter. The handle assembly includes a proximal handle, a rotational handle, and a wire spool. The proximal handle is disposed at a proximal end of the handle assembly, and the proximal handle comprises a manifold comprising at least one side port and a through bore. The wire spool is disposed inside the rotational handle, and the wire spool is configured to wrap a distal portion of a sheath circumferentially along the wire spool. The rotational handle is mechanically coupled with the wire spool, wherein when the rotational handle rotates toward a first rotational direction, the wire spool is configured to wrap the distal portion of the sheath, so that the sheath is pulled to move distally.

The present disclosure describes another embodiment of a method for delivering a pre-loaded stent graft. The method includes disposing a handle assembly at a distal end of a guide wire catheter. The guide wire catheter includes a guide wire lumen therethrough, and the handle assembly includes a proximal handle, a rotational handle, a slot, and a sheath follower. The proximal handle is disposed at a proximal end of the handle assembly, and the proximal handle comprises a manifold comprising at least one side port and a through bore. The slot is disposed inside the rotational handle, and the slot is fixed and remains stationary relative to the proximal handle. The sheath follower is disposed slidably in the slot, and the sheath follower is configured to fix to a distal portion of a sheath. The rotational handle comprises an internal thread mechanically coupled with the sheath follower, so that, when the rotational handle rotates toward a first rotational direction, the sheath is pulled to move distally.

### BRIEF DESCRIPTION OF THE DRAWINGS

FIG. 1 is a schematic diagram of a pre-loaded stent graft delivery device.
FIG. 2 is schematic diagram of a pre-loaded stent graft delivery device including two side ports.
FIG. 3 is a schematic diagram of a pre-loaded stent graft delivery device including three side ports.
FIG. 4 is a schematic diagram of a wire-split sheath.
FIGS. 5A-5E are schematic diagrams of embodiments of a pre-loaded stent graft delivery device.
FIG. 6 is a schematic diagram of another embodiment of a pre-loaded stent graft delivery device.

### DETAILED DESCRIPTION OF THE DRAWINGS

The present disclosure will now be described in detail hereinafter with reference to the accompanied drawings, which form a part of the present disclosure, and which show, by way of illustration, specific examples of embodiments. Please note that the disclosure may, however, be embodied in a variety of different forms and, therefore, the covered or claimed subject matter is intended to be construed as not being limited to any of the embodiments to be set forth below. Please also note that the invention may be embodied as methods, devices, components, or systems. Accordingly, embodiments of the disclosure may, for example, take the form of hardware, software, firmware or any combination thereof.

Throughout the specification and claims, terms may have nuanced meanings suggested or implied in context beyond an explicitly stated meaning. Likewise, the phrase "in one embodiment/implementation" or "in some embodiments/implementations" as used herein does not necessarily refer to the same embodiment/implementation and the phrase "in another embodiment/implementation" or "in other embodiments/implementations" as used herein does not necessarily refer to a different embodiment/implementation. It is intended, for example, that claimed subject matter includes combinations of exemplary embodiments/implementations in whole or in part.

In general, terminology may be understood at least in part from usage in context. For example, terms, such as "and", "or", or "and/or," as used herein may include a variety of meanings that may depend at least in part upon the context in which such terms are used. Typically, "or" if used to associate a list, such as A, B or C, is intended to mean A, B, and C, here used in the inclusive sense, as well as A, B or C, here used in the exclusive sense. In addition, the term "one or more" or "at least one" as used herein, depending at least in part upon context, may be used to describe any feature, structure, or characteristic in a singular sense or may be used to describe combinations of features, structures or characteristics in a plural sense. Similarly, terms, such as "a", "an", or "the", again, may be understood to convey a singular usage or to convey a plural usage, depending at least in part upon context. In addition, the term "based on" or "determined by" may be understood as not necessarily intended to convey an exclusive set of factors and may, instead, allow for existence of additional factors not necessarily expressly described, again, depending at least in part on context.

The present disclosure relates to a pre-loaded stent graft delivery device, which may solve or alleviate the drawbacks as previously discussed. The present disclosure may allow shorter preloaded catheters being used, for example but not limited to, pre-loaded catheters with a length between about 55 cm and about 65 cm, inclusive. The present disclosure may be easier to torque and track during medical procedures compared with existing devices, thus decreasing operation time and/or radiation exposure, and improving medical treatments. Here, "about" a value may refer to a range of ± 5 cm around the value.

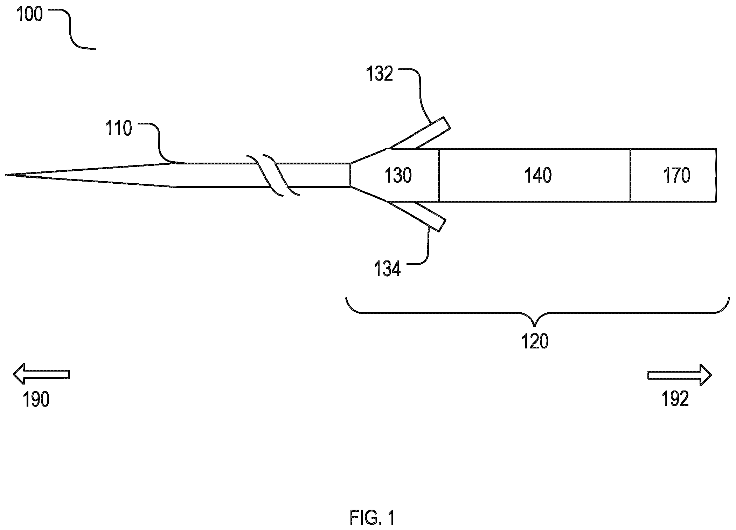

Refering to FIG. 1, the present disclosure describes a pre-loaded stent graft delivery device. The pre-loaded stent graft delivery device 100 may include a guide wire catheter 110 and a handle assembly 120. The pre-loaded stent graft delivery device 100 has a proximal end/direction 190 and a distal end/direction 192.

The guide wire catheter 110 may have a guide wire lumen therethrough, so that one or more sheath may be disposed inside the guide wire lumen, extending from a proximal end of the guide wire catheter to a distal end of the guide wire catheter. In one implementation, a stent graft may be retained within a stent-graft sheath and may be pre-loaded in a proximal region of the guide wire catheter. The stent-graft sheath may be dispoed inside the guide wire lumen and extend from the stent graft to the handle assembly 120. The stent graft may be free from the constrain from the stent-graft sheath when the stent-graft sheath moves a certain distance relative to the stent graft towards the distal direction 192. The stent graft may include a fenestrated stent graft or a branched stent graft.

In another implementation, the guide wire catheter 110 may include more than one guide wire lumens, for example but not limited to, two lumens, three lumens, and four lumens. One or more sheaths may be disposed in the more than one guide wire lumens. For example, each guide wire lumen contains one sheath. For another example, one guide wire lumen contains two sheaths and another guide wire lumen contains one sheath.

In the present disclsoure, the term "stent graft" may be used when referring to a device which has a tubular body of biocompatible graft material and at least one stent fastened to the tubular body to define a lumen through the stent graft. The stent graft may be bifurcated and have fenestrations, side arms or the like. Other arrangements of stent grafts are also within the scope of the present disclsoure.

In the present disclosure, the term "proximal end" (as 190 in FIG. 1) may be used when referring to a part of a delivery device that is furthest from an operator (for example, a physician or other health care provider) and is intended for insertion in a patient's body. For example, a proximal end of a delivery device may refer to an end of the delivery device closest to the heart after placement in the human body of the patient. The term "distal end" is used when referring to that end opposite the proximal end, or a part of a device closest to the operator.

Referring to FIG. 1, the handle assembly 120 may be disposed at a distal end of the guide wire catheter. The handle assembly 120 may include a proximal handle 130 and a rotational handle 140.

The proximal handle 130 may be disposed at a proximal end of the handle assembly. The proximal handle 130 may include a manifold comprising at least one side port and a through bore. The at least one side port may extend distally from the the through bore. For example, the manifold may include two, three, four, five, or six side ports. In one implementation, a catheter or a wire may be pre-loaded through one of the side ports and in its through bore, and another catheter, which may be a same type or different type, may be pre-loaded through another side port. In another implementation, one or more wires may be preloaded into a single side port.

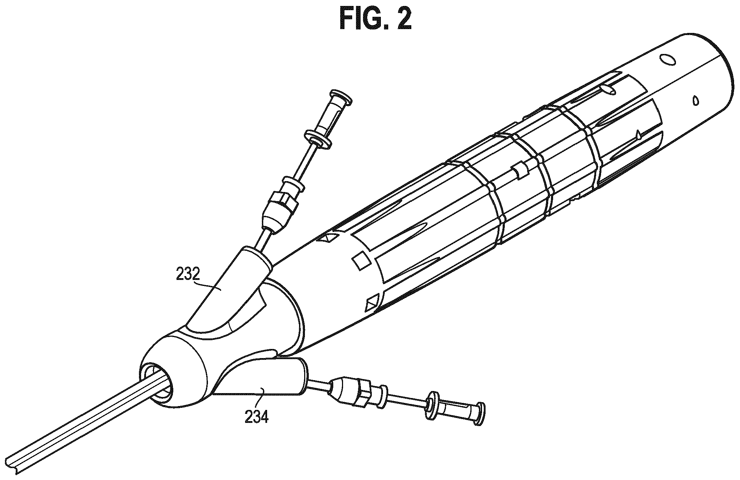

In one implementation, the manifold may include two side ports. The locations of the two side ports may be determined according to a specific medical application. For example, the two side ports may be disposed opposite to each other, i.e, about 180 degree between them, as shown as 132 and 134 in FIG. 1. For another example, the two side ports may be substantially close to each other (as 232 and 234 in FIG. 2), so that an operator may easily access the two side ports simultaneously, for instance, when the operator handles the two side ports with one hand. For example but not limited to, the two side ports may be disposed by about 60 degrees between them. Different catheters may be pre-loaded through one or both of the side ports. Here, "about" a value may refer to a range of ± 10 degrees around the value.

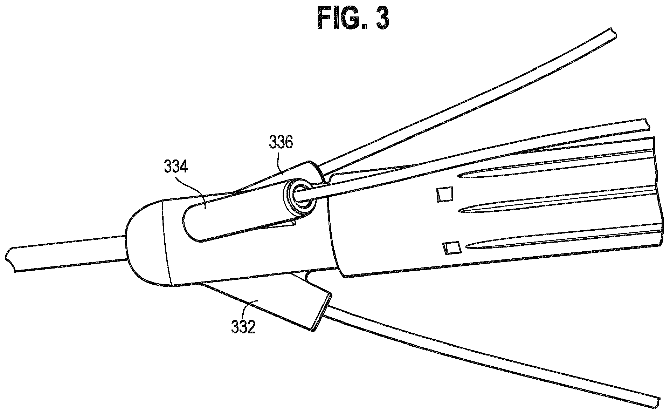

In another implementation, the manifold may include three side ports (as 332, 334, and 336 in FIG. 3). For one example, the three side ports may be disposed evenly around the manifold, i.e., about 120 degrees between each other. In another example, the three side ports may be substantially close to each other, so that an operator may easily access the three side ports simultaneously, for instance, when the operator handles the three side ports with one hand. Different or same types of catheters may be pre-loaded through one or more of the side ports.

Optionally, the handle assembly 120 may include a back handle 170. The back handle may include one or more trigger wire release.

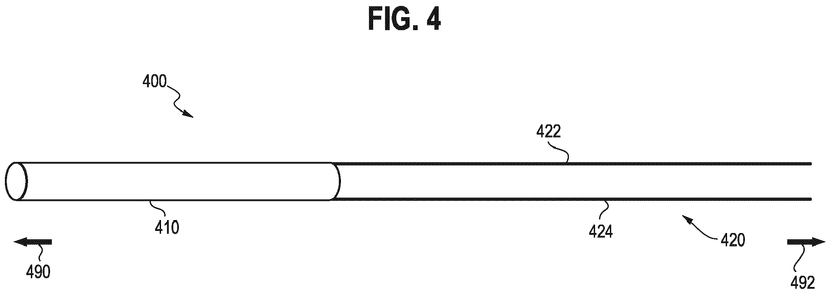

FIG. 4 shows one embodiment of a sheath, which is configured to be disposed in the guide wire lumens. The sheath 400 may include a split-wire sheath, which includes a proximal portion 410 at a proximal direction 490 of the sheath, and a distal portion 420 of the sheath at a distal direction 492 of the sheath. The proximal portion 410 may include a sheath lumen, and a stent graft may be configured to be disposed inside the sheath lumen. When the stent graft is inside the sheath lumen, the sheath may constrain the stent graft; and when the sheath moves a certain distance relative to a distal direction 492, the stent graft may be released from the sheath lumen and may be free to expand to a designed dimension.

The proximal portion 410 may include a circular cross-section shape. In another implementaton, the proximal portion 410 may include an elliptical cross-section shape.

In one implementation, the distal portion 420 of the sheath may be split into a number of pieces evenly, including but not limited to, two, three, four, five and six pieces. For example, the distal portion 420 of the sheath may be split into two pieces, and each piece includes about a half of a circumference of the sheath. For another example, the distal portion 420 of the sheath may be split into three pieces, and each piece includes about a third of a circumference of the sheath. For another example, the distal portion 420 of the sheath may be split into four pieces, and each piece includes about a fourth of a circumference of the sheath. Here, "about" a value may refer to a range from 90 percent of the value to 110% of the value.

In another implementation, the sheath may be split into a number of pieces unevenly. For example, the distal portion 420 of the sheath may be split into three pieces unevenly: one piece includes about a half of a circumference of the sheath; each of the other two pieces includes a fourth of the circumference of the sheath.

In another embodiment, the distal portion 420 of the sheath may include a number of wires, including but not limited to, one, two, three, four, five, and six wires. The one or more wires may embed into the proximal portion 410 and expose a certain length to a distal direction 492. For example, as shown in FIG. 4, the distal portion 420 of the sheath may include two wires (422 and 424), which are about 180 degree between them. Proximal ends of the two wires (422 and 424) may embed into and fix to the proximal portion 410, and be exposed at the distal direction 492.

The one or more wires may include a same or a different cross-section shape, including but not limited to, square, rectangular, circular, elliptical, and triangle shapes.

In another embodiment, the distal portion 420 of the sheath may include a number of strings or threads, including but not limited to, one, two, three, four, five, and six strings or threads. The one or more strings or threads may connect to the proximal portion 410 and expose a certain length towards the distal direction 492. The one or more strings or threads may include a same or a different cross-section shape, including but not limited to, square, rectangular, circular, elliptical, and triangle shapes.

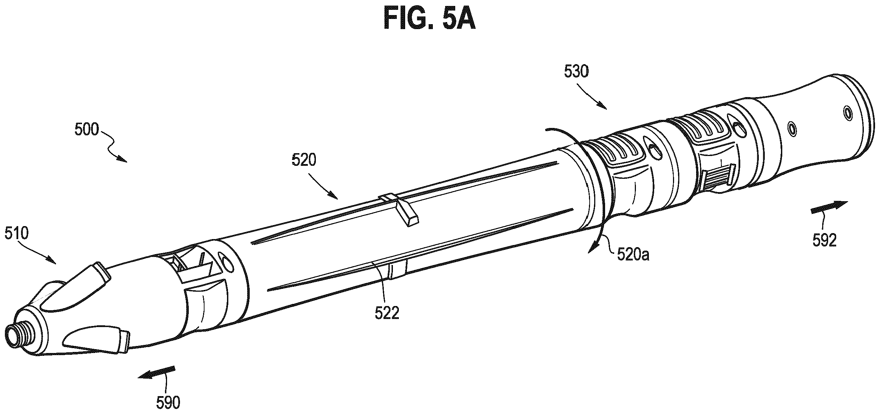

FIG. 5A shows a handle assembly. The handle assembly 500 may include a proximal handle 510, a rotational handle 520, and a back handle 530. The rotational handle 520 may include one or more protrusion 522 on the outer surface. The protrusions 522 are configured to provide friction when an operator rotates the rotational handle. The protrusion 522 may elongate along a longitudinal axis of the handle assembly, which is along a proximal direction 590 and a distal direction 592. The rotational direction may include a courter-clock rotational direction 520a, which is a counter-clock wise rotation to the operator when a distal end of the handle assembly 500 faces towards the operator.

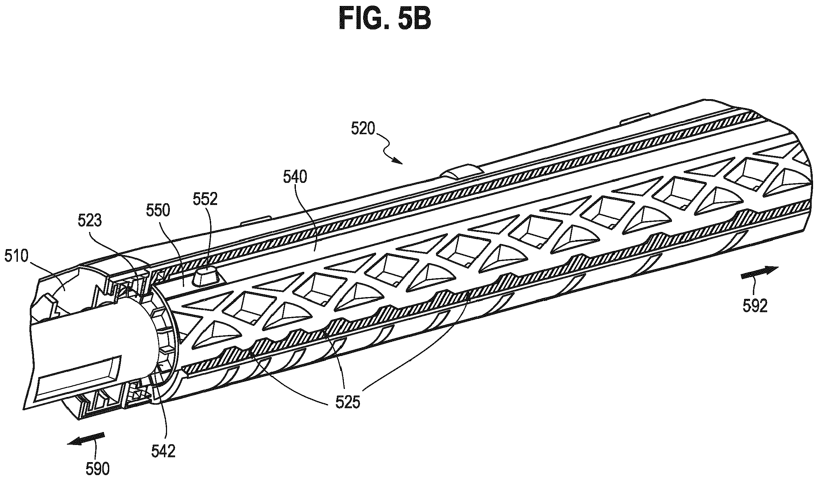

FIG. 5B shows an interior structure of the handle assembly 500. The rotational handle 520 may include an internal thread 525. The internal thread 525 forms a spiral thread track, which is mechanically coupled with a protrusion 552 of a sheath follower 550. The sheath follower 550 is disposed inside a slot 540 and slidable along the longitudinal axis of the rotational handle 520. The slot 540 may be disposed inside the rotational handle 520, and may be mechanically coupled and fixed with the proximal handle 510. When the rotational handle 520 rotates, the slot 540 may remain stationary relative to the proximal handle 510.

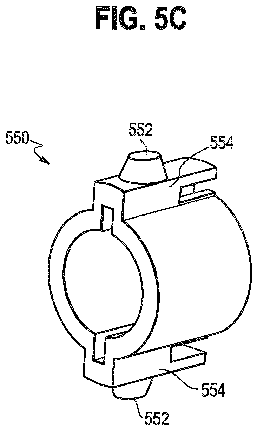

FIG. 5C shows one embodiment for a sheath follower 550. The sheath follower may include a first protrusion portion 554. The first protrusion portion may fit inside the slot 540 and is slidable along the slot 540. The sheath follower 550 may include more than one first protrusion portions 554. For example, the sheath follower 550 includes two first protrusion portions as in FIG. 5C, which are disposed opposite and about 180 degrees to each other.

Referring to FIG. 5C, the sheath follower 550 may include a second protrusion portion 552. The second protrusion portion may be mechanically engaged with a track groove of the internal thread 525 of the rotational handle. When the rotational handle 520 rotates, the sheath follower is configured to move along the proximal-distal axis. In one implementation, the internal thread may be configured to translate the sheath follower towards the distal direction 592 when the rotational handle rotates in the counter-clockwise rotational direction 520a.

In another implementation, the internal thread may be configured to translate the sheath follower towards the distal direction 592 when the rotational handle rotates in a clockwise rotational direction. This implementation may be convenient for certain operators, for example, those who prefer a clockwise rotational direction, and/or those who are left-handed.

Referring to FIG. 5B, the handle assembly may include a first portion of a ratchet mechanism 542, which is configured to couple with a second portion of the ratchet mechanism 523. The first portion of the ratchet mechanism 542 may be fixed to the proximal handle 510. The second portion of the ratchet mechanism 523 may be fixed to the rotational handle 520. When the rotational handle 520 rotates, the first portion of the ratchet mechanism 542 may remain stationary relative to the proximal handle 510, and the second portion of the ratchet mechanism 523 may rotate with the rotational handle 520 around the longitudinal axis of the handle assembly. In one implementation, the first portion of the ratchet mechanism 542 may include a round gear with teeth 542, and the second portion of the racket mechanism 523 may include a pivoting, spring-loaded finger 522. The finger 522 is configured to engage the teeth 542.

In one implementation, when the rotational handle 520 rotates in a counter-clock wise direction, the finger 522 may move in an unrestricted direction around the teeth 542, so that the sheath follower 550 may move distally; and when the rotational handle stops rotating, the finger 522 may engage with the teeth 542 to prevent the rotational handle 520 from rotating in a clock wise direction, so that the sheath follower 550 is prevented from moving proximally.

In one implementation, the internal thread 525 of the rotational handle 520 may have a constant pitch, wherein the sheath follower may move a constant distance along the slot 540 for each turn of rotating the rotational handle. The constant pitch may be in a range between 5 mm per turn and 30 mm per turn, inclusive.

In another implementation, the internal thread 525 of the rotational handle 520 may have a variable pitch, wherein the sheath follower may move a variable distance along the slot 540 for each turn of rotating the rotational handle. For one example as shown in FIG. 5B, a pitch of the internal thread 525 may be smaller at a proximal end than a pitch at a distal end. The smaller pitch at the proximal end may be beneficial to provide a more precise movement control of the sheath follower during a beginning stage of the sheath follower's movement. The smaller pitch at the proximal end may also requires a smaller rotational force to rotate the rotational handle, thus making it easier for the operator to rotate the rotational handle during a beginning stage of the sheath follower's movement.

In one implementation, the pitch of the internal thread 525 may gradually increase from the proximal end to the distal end. For example, the pitch of the internal thread 525 at the proximal end may be between 5 mm and 10 mm per turn; the pitch of the internal thread 525 may gradually increase from the proximal end to the distal end; and the pitch of the internal thread 525 at the distal end may be between 10 mm and 30 mm per turn.

In another implementation, the pitch of the internal thread 525 for the first few turns at the proximal end may be constant, and then the pitch may gradually increase towards the distal end. For example, the pitch of the internal thread 525 for the first three turns at the proximal end may be between 5 mm and 10 mm per turn; the pitch of the internal thread 525 may gradually increase from the fourth turn towards the distal end; and the pitch of the internal thread 525 at the distal end may be between 10 mm and 30 mm per turn.

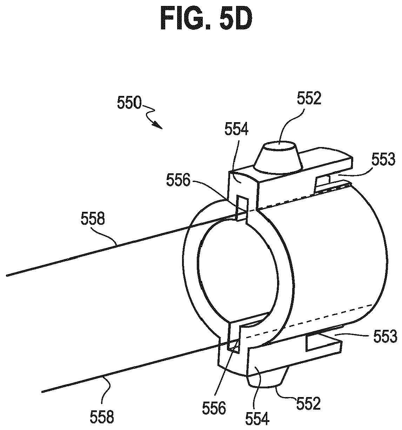

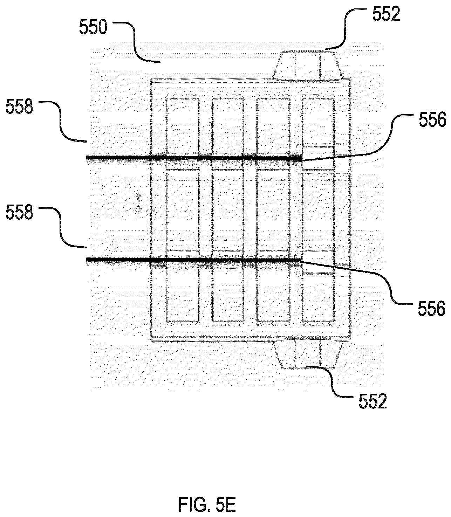

Referring to FIGS. 5D and 5E, the sheath follower 550 may connect to a distal portion 558 of a sheath, wherein the sheath may be a wire-split sheath. The sheath follower 550 may include a notch 556, where the distal portion 558 of the wire-split sheath is fixed to. The fixation between the distal portion 558 and the sheath follower 550 may include, for example but not limited to, ultrasonic welding, heat shrink adhesive, glue, wire bushings, set screws, and other existing fixation methods. For example, referring to FIG. 5D, the sheath follower 550 may include a bushing slot 553 where a bushing is disposed to fix the distal portion 558 of the wire-split sheath to the sheath follower 500.

Referring to FIGS. 5A-5E, when the rotational handle 520 rotates, the sheath follower 550 may move distally and pull the distal portion 558 of the wire-split sheath, so that the wire-split sheath moves distally and exposes a stent graft contained inside a lumen of the wire-split sheath. The stent graft may include a fenestrated stent graft. Optionally, when the stent graft is unsheathed and exposed, a preloaded catheter may be advanced into fenestrations through the distal portion 558 of the wire-split sheath.

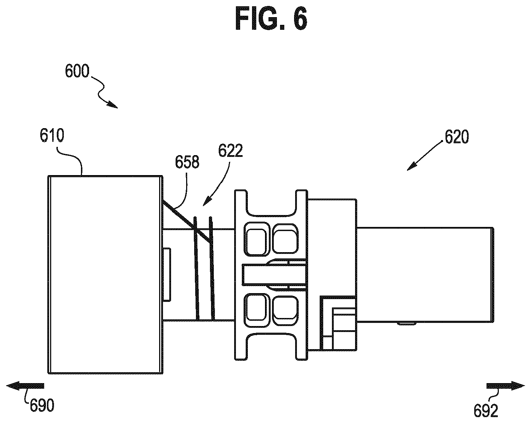

Referring to FIG. 6, the present disclosure describes another embodiment of a rotational handle 620 in a handle assembly 600 in a pre-loaded stent graft delivery device. The rotational handle 620 may be disposed at a distal end of a proximal handle 610. The rotational handle 620 may include a wire spool 622. The wire spool 622 is configured to pull a distal portion 658 of a sheath towards a distal direction 692 by wrapping the distal portion 658 circumferentially along the wire spool 622. The distal portion 558 of the sheath may be fixed to one circumferential point on the wire spool 622. The fixation between the distal portion 658 and the wire spool 622 may include, for example but not limited to, ultrasonic welding, heat shrink adhesive, glue, wire bushings, set screws, and other existing fixation methods.

In one implementation, the wire spool may freely rotate along a longitudinal axis of the handle assembly 600, wherein the longitudinal axis is along a proximal direction 690 and a distal direction 692. The wire spool 622 may be mechanically coupled with the rotational handle 620 so that when the rotational handle rotates, the wire spool may rotate. For one example, the wire spool 622 may be directly fixed to the rotational handle 620, so that the wire spool 622 rotates one turn for every turn of rotating the rotational handle 620. For another example, the wire spool 622 may be mechanically coupled to the rotational handle 620 via a set of gears, so that the wire spool 622 rotates one turn for every two or three turns of rotating the rotational handle 620. In another implementation, the wire spool may be fixed within the rotational handle, so that the wire spool may rotate only when the rotational handle rotates.

In summary, the disclosure is directed to a pre-loaded stent graft delivery device. The pre-loaded stent graft delivery device includes a guide wire catheter having a guide wire lumen therethrough; and a handle assembly disposed at a distal end of the guide wire catheter. The handle assembly includes a proximal handle, a rotational handle, a slot, and a sheath follower. The proximal handle, including a manifold including at least one side port, is disposed at a proximal end of the handle assembly. The slot is disposed inside the rotational handle, and is fixed and remains stationary relative to the proximal handle. The sheath follower is disposed slidably in the slot and fixed to a distal portion of a sheath. The rotational handle includes an internal thread mechanically coupled with the sheath follower, so that, when the rotational handle rotates toward a first rotational direction, the sheath is pulled to move distally.

While the particular invention has been described with reference to illustrative embodiments, this description is not meant to be limiting. Various modifications of the illustrative embodiments and additional embodiments of the invention will be apparent to one of ordinary skill in the art from this description. Those skilled in the art will readily recognize that these and various other modifications can be made to the exemplary embodiments, illustrated and described herein, without departing from the spirit and scope of the present invention. It is therefore contemplated that the appended claims will cover any such modifications and alternate embodiments. Certain proportions within the illustrations may be exaggerated, while other proportions may be minimized. Accordingly, the disclosure and the figures are to be regarded as illustrative rather than restrictive.

## Claims

1. A pre-loaded stent graft delivery device comprising:
a guide wire catheter having a guide wire lumen therethrough; and
a handle assembly disposed at a distal end of the guide wire catheter, the handle assembly comprising a proximal handle, a rotational handle, a slot, and a sheath follower, wherein:
the proximal handle is disposed at a proximal end of the handle assembly, and the proximal handle comprises a manifold comprising at least one side port and a through bore,
the slot is disposed inside the rotational handle, and the slot is fixed and remains stationary relative to the proximal handle,
the sheath follower is disposed slidably in the slot, and the sheath follower is configured to fix to a distal portion of a sheath, and
the rotational handle comprises an internal thread mechanically coupled with the sheath follower, so that, when the rotational handle rotates toward a first rotational direction, the sheath is pulled to move distally.

2. The pre-loaded stent graft delivery device according to claim 1, wherein:
the at least one side port extends distally from the through bore.

3. The pre-loaded stent graft delivery device according to claim 1 or 2, wherein:
a number of side ports of the manifold is between two and four, inclusive.

4. The pre-loaded stent graft delivery device according to any of claims 1 to 3, wherein:
the sheath comprises a split-wire sheath disposed through a through bore within the manifold; and
the split-wire sheath comprises a proximal portion and a distal portion.

5. The pre-loaded stent graft delivery device according to claim 4, wherein:
the sheath follower is configured to fix to the distal portion of the split-wire sheath by at least one of the following process: ultrasonic welding, heat shrink adhesive, glue, wire bushing, and set screw.

6. The pre-loaded stent graft delivery device according to claim 4 or 5, wherein:
the proximal portion of the split-wire sheath comprises a circular or elliptical cross-section shape.

7. The pre-loaded stent graft delivery device according to any of claims 1 to 6, wherein:
the internal thread is rotatable together with the rotation handle along a distal-proximal axis of the handle assembly; and/or
the internal thread comprises a constant pitch.

8. The pre-loaded stent graft delivery device according to any of claims 1 to 7, wherein:
the sheath follower comprises a protrusion engaged with a track groove of the internal thread.

9. The pre-loaded stent graft delivery device according to any of claims 1 to 8, wherein:
the internal thread comprises a variable pitch, where a pitch at a proximal end of the internal thread is smaller than a pitch at a distal end of the internal thread.

10. The pre-loaded stent graft delivery device according to any of claims 1 to 9, wherein:
the first rotational direction is a counter-clock rotational direction when viewing from the distal end of the handle assembly to the proximal end of the handle assembly.

11. A pre-loaded stent graft delivery device comprising:
a guide wire catheter having a guide wire lumen therethrough; and
a handle assembly disposed at a distal end of the guide wire catheter, the handle assembly comprising a proximal handle, a rotational handle, and a wire spool, wherein:
the proximal handle is disposed at a proximal end of the handle assembly, and the proximal handle comprises a manifold comprising at least one side port and a through bore,
the wire spool is disposed inside the rotational handle, the wire spool is configured to wrap a distal portion of a sheath circumferentially along the wire spool, and
the rotational handle is mechanically coupled with the wire spool, wherein when the rotational handle rotates toward a first rotational direction, the wire spool is configured to wrap the distal portion of the sheath so that the sheath is pulled to move distally .

12. The pre-loaded stent graft delivery device according to claim 11, wherein:
the at least one side port extends distally from the through bore; and / or
a number of side ports of the manifold is between two and four, inclusive.

13. The pre-loaded stent graft delivery device according to claim 11 or 12, wherein:
the sheath comprises a split-wire sheath disposed through a through bore within the manifold;
the split-wire sheath comprises a proximal portion and a distal portion.

14. The pre-loaded stent graft delivery device according to claim 13, wherein:
the proximal portion of the split-wire sheath comprises a circular or elliptical cross-section shape.

15. The pre-loaded stent graft delivery device according to any of claims 11 to 14, wherein:
the wire spool is fixed within the rotational handle, so that when the rotational handle rotates, the wire spool rotates; and / or
the first rotational direction is a counter-clock rotational direction when viewing from the distal end of the handle assembly to the proximal end of the handle assembly.
